# EUROPÄISCHE PATENTANMELDUNG

(11) **EP 1 477 473 A1**
(43) Veröffentlichungstag der Anmeldung: **17.11.2004**
(21) Anmeldenummer: 04010421.8
(22) Anmeldetag: 03.05.2004
(51) Int. Cl.: C07C 217/28, C07C 233/36, C09D 5/02, C09D 1/02, C08G 65/333

(54) **Aminoalkohol-basierte Tenside mit geringer Oberflächenspannung und deren Verwendung**

(30) Priorität: 14.05.2003 DE 10321536
(71) Anmelder: Goldschmidt AG, 45127 Essen (DE)
(72) Erfinder: Blanda, Gaetano, Dr., 20090 Segrate-Milano (IT); Eissmann, Ingrid, 45884 Gelsenkirchen (DE); Lehmann, Kathrin, 51377 Leverkusen (DE); Lüther, Heike, 45473 Mülheim (DE); Schunicht, Christoph, Dr., 45147 Essen (DE); Silber, Stefan, Dr., 47804 Krefeld (DE); Tomuschat, Philipp, 45127 Essen (DE)

(57) **Zusammenfassung**

Gegenstand der Erfindung sind Aminoalkohol-basierte Tenside mit geringer Oberflächenspannung und deren Verwendung als Tensid in wässrigen Beschichtungsformulierungen, herstellbar durch Umsetzung mindestens eines sekundären Amins gemäß Formel (I): 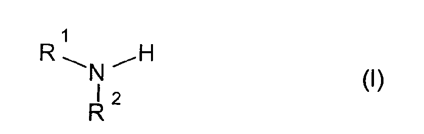 mit mindestens einem Diepoxid gemäß Formel (III): 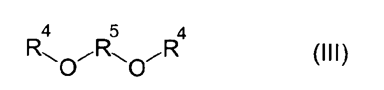 und/oder mindestens einem Triepoxid gemäß Formel (IV): 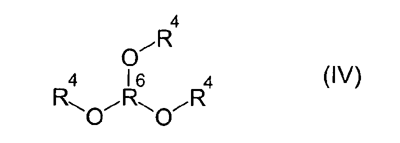 in im wesentlichen äquivalenten Mengen von Aminwasserstoffatomen und Epoxidgruppen.

## Beschreibung

Gegenstand der Erfindung sind Aminoalkohol-basierte Tenside mit geringer Oberflächenspannung und deren Verwendung als Tensid in wässrigen Beschichtungsformulierungen.

Wasserbasierte Farben und Lacke werden in großem Maßstab industriell verwendet. Maßgeblich für eine gute Benetzung des Substrates ist es, dass die Oberflächenspannung des wässrigen Systems mit Hilfe eines Tensids abgesenkt wird. Dabei ist es nicht nur entscheidend, dass die statische Oberflächenspannung auf einen niedrigen Wert abgesenkt wird, sondern auch die dynamische. Insbesondere ist eine geringe dynamische Oberflächenspannung für Hochgeschwindigkeits-Applikationen erforderlich, z.B. beim Aufsprühen von Beschichtungen oder im Bereich von Druckprozessen. Darüber hinaus dürfen die verwendeten Tenside die Ausbildung eines gleichmäßigen Filmes nicht stören, keine Trübung verursachen und sie sollten schwach schäumend sein, d. h. den Aufbau großer Mengen von Schaum nicht begünstigen.

Nichtionische Tenside wie z.B. Alkylaryl- oder Alkohol-Ethoxylate oder Ethylenoxid (EO)-Propylenoxid (PO)-Copolymere sind zwar in der Lage, die statische Oberflächenspannung hervorragend zu reduzieren, jedoch ist es mit diesen Verbindungsklassen, aufgrund ihres hohen Molekulargewichtes und der daraus resultierenden geringen Molekülbeweglichkeit nicht möglich, die dynamische Oberflächenspannung auf einen für den Anwender akzeptablen Wert zu verringern.

Im Gegensatz dazu vermögen einige anionische Tenside wie die Natriumsalze von Mono- oder Dialkylsulfosuccinaten zwar die dynamische Oberflächenspannung gut zu reduzieren, ihre Verwendung führt jedoch zu einem starken Aufbau von Schaum während der Applikation und die fertige Beschichtung reagiert empfindlich auf Wasser.

In jüngerer Zeit kam es zur Entwicklung einer neuen Klasse von Tensiden, die auf acetylenischen Glykolen und ihren Alkoxylaten beruhen. Die Eigenschaften dieser Tenside liegen zwischen denen der zuvor geschilderten Tenside. Mit diesen Tensiden ist es möglich, sowohl die statische als auch die dynamische Oberflächenspannung zu reduzieren, wobei die erreichbaren Werte nicht ganz an die der nichtionischen und anionischen heranreichen. Dafür sind Formulierungen mit diesen Tensiden vergleichsweise schaumarm (EP-B-0 897 744, US-2 997 447).

Aufgrund dieser Eigenschaften haben sich derartige Tenside in zahlreichen Anwendungen etablieren und durchsetzen können. Dabei werden die Eigenschaften hauptsächlich zurückgeführt auf den starren acetylenischen Alkylspacer, der dafür Sorge trägt, dass es durch die eingeschränkten Freiheitsgrade zu einer Art Vororientierung von polaren und unpolaren Gruppen kommt. Darüber hinaus wurde der geringe Abstand der polaren Gruppen und das geringe Molekulargewicht (< 300 g/mol), das eine hohe Beweglichkeit der Tensidmoleküle zulässt, für die Eigenschaften verantwortlich gemacht.

Problematisch an Verbindungen diesen Typs ist, dass es in Anwendungen nach recht kurzer Zeit erneut zu einem Schaumaufbau kommt. Für den Anwender ist es jedoch von großer Bedeutung, diesen Neuaufbau über einen möglichst langen Zeitraum zu verhindern. Andernfalls müssen Entschäumer zudosiert werden, die unter anderem zu unerwünschten Störungen in der Ausbildung des Lackfilms und Problemen bei der Zwischenlagenhaftung führen können.

Es bestand daher der Bedarf, Verbindungen zur Verfügung zu stellen, die sowohl eine gute Reduktion der statischen und dynamischen Oberflächenspannung ermöglichen, als auch den Aufbau/Neuaufbau von Schaum über einen langen Zeitraum wirkungsvoll verhindern.

In dem Bestreben, die Nachteile des Standes der Technik zu überwinden und Verbindungen bereitzustellen, die die dynamische Oberflächenspannung deutlich reduzieren und gleichzeitig die Bildung/Neubildung von Schaum über einen langen Zeitraum effektiv inhibieren, wurde nun überraschenderweise gefunden, dass dieses Ziel durch Aminoalkohole, herstellbar durch Umsetzung von mindestens ein Aminwasserstoff enthaltenden, vorzugsweise sekundären Aminen, mit Glycidylverbindungen erreicht werden kann.

Ein Gegenstand der Erfindung sind daher Aminoalkohole erhalten durch die Umsetzung eines oder mehrerer Amine gemäß Formel (I) 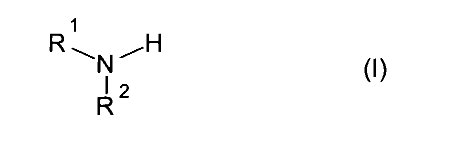 worin
- R¹ und R²: unabhängig voneinander ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen oder eine Gruppierung der allgemeinen Formel (II) 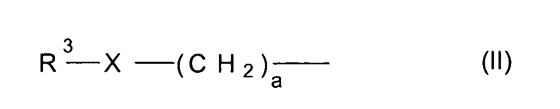 worin
R³ ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen,
X ein Rest aus der Gruppe -O-C(O)-, -(O)C-O-, - NH-C (O) -, - (O) C-NH, - (CH₃) N-C (O) -, - (O) C-N (CH₃) -, - S (O₂) -O-, -O-S (O₂) -, -S (O₂) -NH-, -NH-S (O₂) -, - S (O₂) -N (CH₃) - , -N (CH₃) - S (O₂) - ,
a eine ganzzahlige Variable zwischen 1 und 4 ist, mit einem Epoxid gemäß Formel (III) 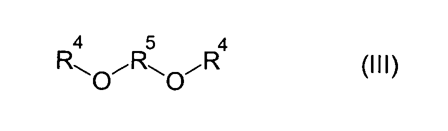 wobei die Reste
R⁴ unabhängig voneinander H oder 2,3-Epoxypropyl sind mit der Maßgabe, dass durchschnittlich mehr als ein, vorzugsweise mehr als anderthalb 2,3-Epoxypropylreste im Molekül enthalten sind,
R⁵ ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen.

Ein weiterer Gegenstand der Erfindung sind Aminoalkohole erhalten durch die Umsetzung eines oder mehrerer Amine gemäß der oben genannten Formel (I) mit einem Epoxid gemäß Formel (IV) 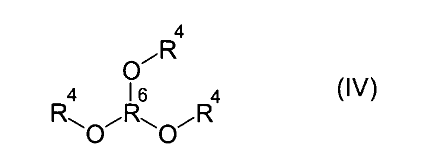 wobei die Reste
- R⁴: unabhängig voneinander H oder 2,3-Epoxypropyl sind mit der Maßgabe, dass durchschnittlich mindestens ca. zwei 2,3-Epoxypropylreste im Molekül enthalten sind,
- R⁶: ist ein gegebenenfalls verzweigter, gegebenenfalleine Hydroxylgruppe oder andere Heteroatomsubstituenten enthaltender, gegebenenfalls ungesättigter Rest mit 1 bis 30 C-Atomen.

Ein weiterer Gegenstand der Erfindung sind Aminoalkohole der allgemeinen Formeln (V) und (VI) 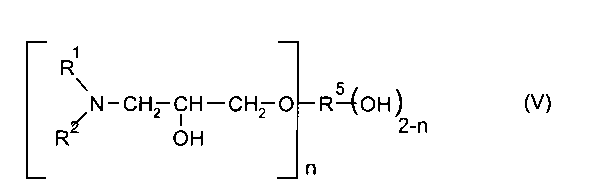 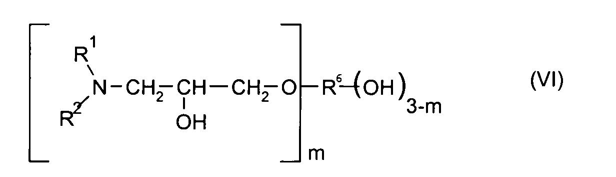 herstellbar gemäß obiger Umsetzung, worin
- R¹, R²: die oben angegebene Bedeutung haben, und unabhängig voneinander vorzugsweise gegebenenfalls verzweigte Alkylenreste mit 1 bis 10, insbesondere 3 bis 6 C-Atomen, und/oder R³-X- (CH₂) ₐ- worin R³, X, a die oben angegebene Bedeutung haben, R³ vorzugsweise ein gegebenenfalls verzweigter Alkylrest mit 1 bis 10, insbesondere 2 bis 8 C-Atomen und X = -(O)C-NH- und a = 3 ist,
- R⁵ und R⁶: die oben angegebene Bedeutung haben, und unabhängig voneinander vorzugsweise gegebenenfalls verzweigte Alkylenreste mit 1 bis 10, insbesondere 3 bis 6 C-Atomen, und
- n: 1 bis 2, vorzugsweise > 1,5 bis 2 und insbesondere ca. 2,
- m: 1 bis 3, vorzugsweise > 1,5 bis 2,5 und insbesondere ca. 2 bedeuten.

Ein weiterer Gegenstand der Erfindung ist die Verwendung der erfindungsgemäßen Aminoalkohole als Oberflächenspannung reduzierende und gleichzeitig die Bildung/Neubildung von Schaum über einen langen Zeitraum effektiv inhibierende Additive in wässrigen Formulierungen für insbesondere Oberflächenbeschichtungen, Anstrichmittel, Druckfarben oder Lacke.

Ein weiterer Gegenstand der Erfindung sind wässrige Formulierungen enthaltend mindestens einen der erfindungsgemäßen Aminoalkohole, wobei üblicherweise derartige Netzmittel in Mengen von 0,05 % bis 5 %, vorzugsweise von 0,1 % bis 3 % eingesetzt werden.

Die erfindungsgemäß verwendeten Amine und Glycidylether sind technische Produkte, die in Form ihrer jeweiligen handelsüblichen Spezifikationen einsetzbar sind, wobei in speziellen Anwendungsgebieten der erfindungsgemäßen Aminoalkohole aber höhere Reinheitsgrade gefordert werden können.

Besonders bevorzugte Reste R¹ und R² im Amin sind n-Propyl-, i-Propyl-, n-Butyl-, i-Butylreste oder die Amidoamine, herstellbar aus kurzkettigen Carbonsäuren mit Aminen der Formel X- (CH₂) ₐ-NH-R¹ oder X- (CH₂) ₐ-NH-R² worin X = NH₂ und a = 2 oder 3 ist.

Als Diglycidylether werden bevorzugt Ethylenglycoldiglycidylether, 1,2-Propandioldiglycidylether, 1,3-Butandioldiglycidylether, 1,4-Butandioldiglycidylether, Neopentylglykoldiglycidylether, Cyclohexandimethanoldiglycidylether, Diethylenglykoldiglycidylether, Dipropylenglykoldiglycidylether, Polyethylenglykoldiglycidylether, Polypropylenglykoldiglycidylether, Glycerindiglycidylether, Resorcindiglycidylether, 2,2-Bis[4-(glycidyloxy)-phenyl]propan, Bis(4-glycidyloxyphenyl)-methan, Bisphenol-A-propoxylat (1-PO/phenol)di-gycidylether, 1,2-Cyclohexandicarbonsäurediglycidylester eingesetzt.

Als Triglycidylether werden vorzugsweise Glycerintriglycidylether, Trimethylolpropan-triglycidylether, Triphenylolmethantriglycidylether verwandt.

Besonders bevorzugte Glycidylverbindungen sind solche mit zwei oder drei funktionellen Gruppen, wie beispielsweise Butandioldiglycidylether, Neopentylglykoldiglycidylether, Trimethylolpropantriglycidylether, Glycerindiglycidylether.

Amine und Glycidylverbindungen werden, bezogen auf reaktive Aminwasserstoffatome und Epoxidgruppen, in vorzugsweise etwa äquivalenten Mengen eingesetzt. Als Berechnungsgrundlage dienen die dem Fachmann geläufigen Werte der Aminzahl und des Epoxidwertes.

### Experimenteller Teil:

Der vollständige Umsatz aller Reaktionen wurde durch ¹H-NMR-Messungen verifiziert.

### Beispiel 1:

### Umsetzung von Butandioldiglycidylether mit Hexansäureamidopropylmonomethylamin

50 g 81 %iges Hexansäureamidopropylmonomethylamin (0,22 mol) werden unter Stickstoff und Rühren auf 50 °C erhitzt. Anschließend werden 23,1 g (0,11 mol) Butandioldiglycidylether langsam zugetropft. Nach beendeter Zugabe wird die Mischung bei 70 °C für 5 h gerührt. Nach beendeter Reaktion wird abgekühlt und man erhält als Produkt eine hochviskose orange Flüssigkeit.

### Beispiel 2:

### Umsetzung von Butandioldiglycidylether mit Diisopropylamin

50 g (0,5 mol) Diisopropylamin werden unter Stickstoff und Rühren auf 50 °C erhitzt. Anschließend werden 49,9 g (0,25 mol) Butandioldiglycidylether langsam zugetropft. Nach beendeter Zugabe wird die Mischung bei 70 °C für 5 h gerührt. Nach beendeter Reaktion wird abgekühlt und man erhält als Produkt eine schwachgelbe Flüssigkeit.

### Beispiel 3:

### Umsetzung von Butandioldiglycidylether mit Di-n-butylamin

50 g (0,39 mol) Di-n-butylamin werden unter Stickstoff und Rühren auf 50 °C erhitzt. Anschließend werden 39,1 g (0,19 mol) Butandioldiglycidylether langsam zugetropft. Nach beendeter Zugabe wird die Mischung bei 70 °C für 5 h gerührt. Nach beendeter Reaktion wird abgekühlt und man erhält als Produkt eine gelbe Flüssigkeit.

### Beispiel 4:

### Umsetzung von Butandioldiglycidylether mit Diisobutylamin

Eine Mischung aus 25,6 g (0,20 mol) Diisobutylamin und 20,0 g (0,10 mol) Butandioldiglycidylether werden unter Stickstoff und Rühren für 10 h auf 120 °C erhitzt. Nach beendeter Reaktion wird abgekühlt und man erhält als Produkt eine gelbe Flüssigkeit.

### Beispiel 5:

### Umsetzung von Butandioldiglycidylether mit Di-n-pentylamin

50 g (0,32 mol) Di-n-pentylamin werden unter Stickstoff und Rühren auf 50 °C erhitzt. Anschließend werden 32,1 g (0,16 mol) Butandioldiglycidylether langsam zugetropft. Nach beendeter Zugabe wird die Mischung bei 70 °C für 8 h gerührt. Nach beendeter Reaktion wird abgekühlt und man erhält als Produkt eine gelbe Flüssigkeit.

### Beispiel 6:

### Umsetzung von Neopentylglykoldiglycidylether mit Di-n-butylamin

Eine Mischung aus 20,5 g (0,16 mol) Di-n-butylamin und 20,0 g 86 %iger Neopentylglykoldiglycidylether (0,08 mol) werden unter Stickstoff und Rühren für 14 h auf 110 °C erhitzt. Nach beendeter Reaktion wird abgekühlt und man erhält als Produkt eine gelbe Flüssigkeit.

### Beispiel 7:

### Umsetzung von Neopentylglykoldiglycidylether mit Diisobutylamin

Eine Mischung aus 20,5 g (0,16 mol) Diisobutylamin und 20,0 g 86 %iger Neopentylgylkoldiglycidylether (0,08 mol) werden unter Stickstoff und Rühren für 14 h auf 120 °C erhitzt. Nach beendeter Reaktion wird abgekühlt und man erhält als Produkt eine gelbe Flüssigkeit.

### Beispiel 8:

### Umsetzung von Glycerindiglycidylether mit Di-n-butylamin

Eine Mischung aus 18,4 g (0,14 mol) Di-n-butylamin und 20,0 g 73 %iger Glycerindiglycidylether (0,07 mol) werden unter Stickstoff und Rühren für 14 h auf 100 °C erhitzt. Nach beendeter Reaktion wird abgekühlt und man erhält als Produkt eine schwach gelbe Flüssigkeit.

### Beispiel 9:

### Umsetzung von Glycerindiglycidylether mit Diisobutylamin

Eine Mischung aus 18,4 g (0,14 mol) Diisobutylamin und 20,0 g 73 %iger Glycerindiglycidylether (0,07 mol) werden unter Stickstoff und Rühren für 12 h auf 100 °C erhitzt. Nach beendeter Reaktion wird abgekühlt und man erhält als Produkt eine schwach gelbe Flüssigkeit.

### Beispiel 10:

### Umsetzung von Polypropylenglykoldiglycidylether mit Di-n-butylamin

Eine Mischung aus 14,1 g (0,11 mol) Di-n-butylamin und 20,0 g Polypropylenglykoldiglycidylether (0,055 mol) werden unter Stickstoff und Rühren für 14 h auf 100 °C erhitzt. Nach beendeter Reaktion wird abgekühlt und man erhält als Produkt eine gelbe viskose Flüssigkeit.

### Beispiel 11:

### Umsetzung von Polypropylenglykoldiglycidylether mit Diisobutylamin

Eine Mischung aus 14,1 g (0,11 mol) Diisobutylamin und 20,0 g Polypropylenglykoldiglycidylether (0,055 mol) werden unter Stickstoff und Rühren für 14 h auf 100 °C erhitzt. Nach beendeter Reaktion wird abgekühlt und man erhält als Produkt eine orange, viskose Flüssigkeit.

### Beispiel 12:

### Umsetzung von Trimethylolpropantriglycidylether mit Di-n-butylamin

Eine Mischung aus 18 g (0,14 mol) Di-n-butylamin und 20,0 g 70 %iger Trimethylolpropantriglycidylether (0,05 mol) werden unter Stickstoff und Rühren für 14 h auf 100 °C erhitzt. Nach beendeter Reaktion wird abgekühlt und man erhält als Produkt eine gelbe viskose Flüssigkeit.

### Beispiel 13:

### Umsetzung von Trimethylolpropantriglycidylether mit Diisobutylamin

Eine Mischung aus 18 g (0,14 mol) Diisobutylamin und 20,0 g 70 %iger Trimethylolpropantriglycidylether (0,05 mol) werden unter Stickstoff und Rühren für 14 h auf 120 °C erhitzt. Nach beendeter Reaktion wird abgekühlt und man erhält als Produkt eine gelbe viskose Flüssigkeit.

### Anwendungstests:

Für die Abprüfung neuer Netzmittel führt ein Fachmann eine Reihe von Tests zur Übersicht durch, um die schauminhibierende bzw. -verhindernde Wirkung ebenso zu beurteilen, wie auch die durch das Tensid initiierte schnelle Schaumzerstörung, wenn sich Schaum durch andere oberflächenaktive Substanzen in einem System gebildet hat. Ein wesentliches weiteres Kriterium zur Einstufung von Surfactants ist deren Langzeitwirkung im Sinne einer Schaumverhinderung auch nach Lagerung des entsprechenden mit dem Netzmittel ausgestatteten Systems.

### Dynamische Oberflächenspannung:

Die Bestimmung der dynamischen Oberflächenspannung der formulierten Systeme ist essentiell, um die Geschwindigkeit abschätzen zu können, mit der ein Netzmittelmolekül an eine neuerlich generierte Grenzfläche gelangt, um dadurch aktiv zur Schaumzerstörung beitragen zu können.

Für die Bestimmung dieser Werte wird das onlinetensiometer t 60 der Firma SITA Messtechnik GmbH verwandt. Durch das Gerät wird die dynamische Oberflächenspannung nach dem Prinzip des maximalen Blasendrucks gemessen: Durch die innere Anziehungskraft einer Flüssigkeit werden auch Luftblasen, die sich in der Flüssigkeit befinden, komprimiert. Der dabei entstehende Druck steigt mit abnehmendem Blasenradius. Den gegenüber der Umgebung erhöhten Druck macht man sich bei der Blasendruckmethode zunutze. Dazu wird durch eine in eine Flüssigkeit getauchte Kapillare ein Gasstrom geleitet. Die sich dabei bildende Blasenoberfläche wölbt sich und verringert dabei kontinuierlich den Blasenradius. Dabei steigt der Druck bis zu einem Maximaldruck. Bei diesem hat die Blase ihren kleinsten Radius, den Kapillarradius, erreicht und bildet eine Halbkugel. Nach Überschreiten dieses Punktes platzt die Blase auf und reißt von der Kapillare ab, so dass sich eine neue Blase bilden kann. Dabei kommt es zu einem charakteristischen Druckverlauf, der zur Bestimmung der Oberflächenspannung ausgewertet wird. D.h. je kleiner der Wert bei geringer Blasenfrequenz, umso effektiver ist das Netzmittel in der Benetzung einer niedrigenergetischen Oberfläche. Je geringer der Unterschied zwischen dem Wert bei niedriger und hoher Blasenfrequenz, desto besser ist das Netzmittel in der Lage sich an neu geschaffene Oberflächen zu orientieren, d.h. auch während hoch dynamischer Applikationsprozesse wirksam zu sein.

Für die Beurteilung der erfindungsgemäß beanspruchten Netzmittel wurden die im folgenden näher ausgeführten Tests durchgeführt.

### Schauminhibierende Wirkung:

Zu einer vorgegebenen Menge eines Testsystems wird eine definierte Menge Netzmittel gegeben und mittels Zahnradscheibe 1 Minute bei 1.500 Upm eingearbeitet. Anschließend wird 1 Minute bei 3.000 Upm Luft eingetragen und Schaum erzeugt. Die resultierende Schaumhöhe wird abgelesen und im Vergleich zur Schaumhöhe ohne Verwendung des Netzmittels betrachtet. Im Anschluss daran wird die Zeit gemessen, bis ein vollständiger Abbau des Schaums stattgefunden hat, der ohne Verwendung von Netzmitteln im Allgemeinen gar nicht stattfindet.

### Beurteilung des Schaumaufbaus und der Spontanentschäumung:

In einer vorgegebenen Menge eines Testsystems wird mittels Lochscheibe (siehe unten) 1 Minute bei 2.000 Upm Schaum aufgebaut. Anschließend wird eine definierte Menge Netzmittel auf den Schaum gegeben und das Auftreten von Spontanentschäumung dabei visuell beurteilt (zerplatzende Luftblasen, "Prickeln" auf der Oberfläche) und mit nicht vorhanden (-), vorhanden (+/-) oder sehr charakteristisch (+) aufgeführt.

Anschließend erfolgt eine erneute einminütige Scherung mit der Lochscheibe bei 2.000 Upm. Dabei wird die Zeit gestoppt, bis ein erneuter Schaumaufbau erfolgt. Kann ein Netzmittel den erneuten Schaumaufbau verhindern, ist es mit "> 60 s" als sehr aktiv einzustufen.

Baut das Netzmittel den Schaum während der Scherung weiter ab, charakterisiert die Einstufung "kein weiterer Schaumaufbau" in Verbindung mit sehr geringen Schaumhöhen das Netzmittel als schaumarm.

Eine definierte Menge dieser Probe wird anschließend in einen Messzylinder gegeben und die Schaumhöhe wird durch die Angabe der ml Schaum festgehalten und vergleichend zur Blindprobe betrachtet.

Bei der verwandten Lochscheibe handelt es sich um drei an einer Achse übereinander angeordnete Scheiben (Dicke 3 mm, Durchmesser 25 mm) mit je drei Löchern (Durchmesser 5 mm) . Der Abstand der Einzelscheiben zueinander beträgt 9 mm und sie weisen an der Befestigungsachse eine vertikale Drehung um 120 ° auf. Dieses Aggregat ermöglicht einen optimalen Eintrag von Makro- und Mikroschaum, wie er bei Lackierapplikationsprozessen (wie z.B. Walzen, Spritzen) und Herstellprozessen auftritt und durch geeignete Netzmittel zu verhindern ist.

### Langzeitwirkung:

Nach einer Lagerung der zweifach schwerbelasteten Probe (siehe oben beschriebener Test) von 4 bis 14 Tagen wird die Probe erneut mit der Lochscheibe, 1' bei 2.000 Upm, gerührt und nochmals die sich ergebende Schaumhöhe einer Probe in einem Messzylinder abgelesen. Weichen diese Werte kaum von der Erstbestimmung ab, steht das Netzmittel weiterhin im System zur Verfügung und zeichnet sich also auch als hydrolysestabil aus.

In den folgenden Tests werden die erfindungsgemäßen Netzmittel mit S1, S2, und S3 bezeichnet.
- S1: Butandioldiglycidylether mit Diisobutylamin (Beispiel 4)
- S2: Neopentyldiglycidylether mit Diisobutylamin (Beispiel 7)
- S3: Glycerindiglycidylether mit Diisobutylamin (Beispiel 9)

Nichterfindungsgemäße Vergleichsbeispiele sind die nachfolgenden Netzmittel, die als handelsübliche Produkte für wässrige Systeme angeboten werden und entsprechend den unten stehenden Angaben charakterisierbar sind.
- V1: 2,4,7,9-Tetramethyl-5-decin-4,7-diol in Ethylenglycol (50 %ige Lösung)
- V2: 2,4,7,9-Tetramethyl-5-decin-4,7-diolethoxylat
- V3: Fettalkoholalkoxylat mit einem Molgewicht von etwa 500 g/mol

Die vorgenannten erfindungsgemäßen und handelsüblichen Netzmittel werden zur Verwendung in den folgenden üblichen Formulierungen herangezogen.

### Wasserbasierte Druckfarbenformulierung:

In 50 g Farbe bestehend aus:
JonCryl® 8085 (43 %ige ammoniakalische Lösung eines Acrylatharzes)¹⁾ 29,4 g
JonCryl® ECO 2189 (glycoletherfreie filmbildende Polymerdispersion)¹⁾ 44,1 g
JonCryl® ECO 2177 (glycoletherfreie filmbildende Polymerdispersion)¹⁾ 17,7 g
JonWax® 35 (Polyethylenwachsemulsion)¹⁾ 4,9 g demineralisiertes Wasser 2,9 g
werden in einer 100 ml Glasflasche eingewogen und mit einer 2,5 cm-Zahnrandscheibe 1' bei 1.500 Upm 0,5 % Wirkstoff Netzmittel eingerührt und anschließend 1' bei 3.000 Upm aufgeschäumt. Die Füllhöhe (Lösung + Schaum) wird mittels Lineal in der Glasflasche abgelesen und die Zeit des Schaumzusammenbruchs in Minuten mittels Stoppuhr bestimmt.
¹⁾ Johnson Polymer

Für die Bestimmung der dynamischen Oberflächenspannung werden auf 48 g Farbe mit 0,5 % Netzmittel 12 g Wasser gegeben. Die Mischung wird durch einfaches Schütteln homogenisiert.

**Tabelle 1:**

| Ergebnisse in einer wasserbasierten Druckfarbe: | | | |
|---|---|---|---|
| **Netzmittel** | **Schaum [cm]** | **Zeit bis zum Schaum- zusammenbruch [min]** | **Dynamische Oberflächenspannung bei 2 und 10 Blasen/sec [mN/m]** |
| Ohne | 7,0 | stabil, > 12 h | 43,0 - 55,7 |
| S1 | 4,7 | 180* | 40,4 - 53,9 |
| S2 | 5,5 | 120* | 41,4 - 52,8 |
| S3 | 5,5 | 60* | 40,4 - 49,4 |
| V1 | 6,0 | stabil, > 12 h | 39,6 - 51,8 |
| V2 | 5,5 | stabil, > 12 h | 42,7 - 55,1 |
| V3 | 6,7 | stabil, > 12 h | 42,7 - 55,0 |

| | | | |
|---|---|---|---|
| *Restmenge von weniger als 1 % der Ursprungsmenge Schaum erreicht | | | |

Tabelle 1 zeigt, dass durch die Verwendung der erfindungsgemäß beanspruchten Netzmittel der Schaumaufbau gegenüber der Blindprobe und den Vergleichsbeispielen reduziert ist. Resultierend daraus kann die erfindungsgemäß beanspruchte Stoffklasse eine vollständige Entschäumung garantieren.

### Wasserbasierter Autolack:

50 g einer Mischung aus 2 Teilen aliphatischer Polyurethan-Acryl-Hybrid-Dispersion Daotan® VTW 6264 (Firma Solutia) und einem Teil VE-Wasser werden eine Minute mit 2.000 Upm in einem Becher (Durchmesser 65 mm) mit einer Lochscheibe (Beschreibung siehe oben) aufgeschäumt. Auf den resultierenden Schaum werden 0,2 % Wirkstoff Netzmittel gegeben und die Spontanentschäumung beobachtet. Anschließend wird erneut 1 Minute bei 2.000 Upm geschert und die Zeit bis zum erneuten Schaumaufbau mittels Stoppuhr ermittelt. Tritt kein erneuter Schaumaufbau auf, ist die Bewertung mit > 60 sec angegeben.

25 g dieser Probe werden im direkten Anschluss an die Scherbelastung in einen 100 ml Messzylinder gegeben und die Füllhöhe in ml abgelesen.

Zur Beurteilung der Hydrolyse- und Lagerstabilität wird die Probe nach vier Tagen erneut 1 Minute bei 2.000 Upm geschert und die Schaumhöhe von 25 g mittels 100 ml Messzylinder ermittelt.

**Tabelle 2:**

| Ergebnisse in einem wässrigen Autolack: | | | | |
|---|---|---|---|---|
| **Netzmittel** | **Spontanentschäumung*** | **Wiederaufbau von Schaum [sec]** | **Schaumwert sofort [ml/25 g]** | **Schaumwert nach 4 Tagen [ml/25 g]** |
| Blindwert | entfällt | entfällt | 44 | 46 |
| S1 | +/- | > 60 | 27 | 29 |
| S2 | +/- | > 60 | 28 | 30 |
| S3 | +/- | > 60 | 29 | 31 |
| V1 | + | 45 | 30 | 32 |
| V2 | +/- | > 60 | 37 | 40 |
| V3 | + | 45 | 30 | 33 |

| | | | | |
|---|---|---|---|---|
| * (-) nicht vorhanden, (+/-) vorhanden, (+) sehr deutlich ausgeprägt | | | | |

Die erfindungsgemäßen Verbindungen zeigen eine gute Spontanentschäumung, wobei durch weiteren Schereintrag eine signifikante Entschäumung erfolgt, wie sie von den Vergleichssubstanzen nicht geleistet werden kann. Dieses enorme Potential der erfindungsgemäßen Verbindungen ist auch nach Lagerung der Systeme vorhanden, so dass weder Netzmittel nachdosiert noch zusätzlicher Entschäumer ein-gesetzt werden muss.

### Fazit:

Die erfindungsgemäß beanspruchte Netzmittelgruppe kann nicht nur als Spontanentschäumer genutzt werden, sondern bei einer Erstausstattung von Proben bei Herstellung der Systeme mit derartigen Netzmitteln ist eine Schauminhibierung auch nach Lagerung und bei späterer Verwendung der Systeme gegeben.

## Patentansprüche

1. Aminoalkohole, herstellbar durch Umsetzung mindestens eines sekundären Amins gemäß Formel (I): 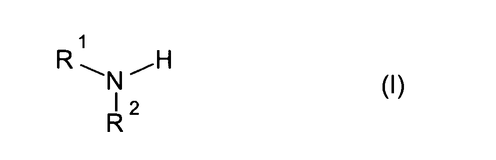 worin
R¹ und R² unabhängig voneinander ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen, eine Gruppierung der allgemeinen Formel (II) 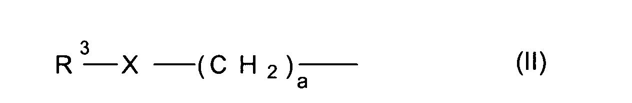 worin
R³ ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen,
X ein Rest aus der Gruppe -O-C(O)-, -(O)C-O-, - NH-C (O) -, - (O) C-NH, - (CH₃) N-C (O) -, - (O) C-N (CH₃) -, -S (O₂) -O-, -O-S (O₂) -, -S (O₂) -NH-, - NH-S(O₂)-, -S(O₂)-N(CH₃)-, -N(CH₃)-S(O₂)-,
a eine ganzzahlige Variable zwischen 1 und 4 ist, bzw. Mischungen der Amine, mit mindestens einem Epoxid gemäß Formel (III): 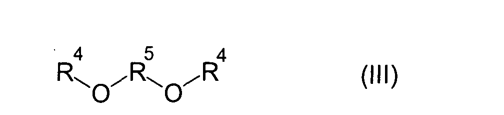
wobei die Reste
R⁴ unabhängig voneinander H oder 2,3-Epoxypropyl sind mit der Maßgabe, dass durchschnittlich mehr als ein 2,3-Epoxypropylrest im Molekül enthalten ist,
R⁵ ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen, und/oder mindestens einem Epoxid gemäß Formel (IV): 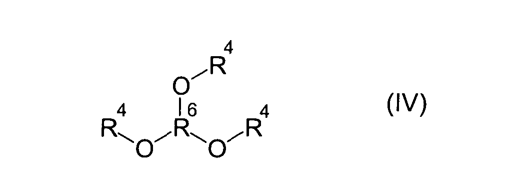
wobei die Reste
R⁴ unabhängig voneinander H oder 2,3-Epoxypropyl sind mit der Maßgabe, dass durchschnittlich mindestens zwei 2,3-Epoxypropylreste im Molekül enthalten sind,
R⁶ ein gegebenenfalls verzweigter, gegebenenfalls eine Hydroxylgruppe oder andere Heteroatomsubstituenten enthaltender, gegebenenfalls ungesättigter Rest mit 1 bis 30 C-Atomen.

2. Verbindungen gemäß Anspruch 1, **dadurch gekennzeichnet, dass** die Reste R¹ und R² des Amins (I) unabhängig voneinander gegebenenfalls verzweigte, gegebenenfalls eine Hydroxylgruppe oder andere Heteroatomsubstituenten enthaltende, gegebenenfalls ungesättigte Reste mit 1 bis 10 C-Atomen sind.

3. Verbindungen gemäß den Ansprüchen 1 und 2, **dadurch gekennzeichnet, dass** das Amin (I) mindestens eine Verbindung ist aus der Gruppe Di-n-Propylamin, Diisopropylamin, Di-n-Butylamin, Di-sec.-butylamin, Di-n-Pentylamin, Diisopentylamin, Dihexylamin, Glycerintriglycidylether, Trimethylolpropantriglycidylether, Triphenylolmethantriglycidylether.

4. Verbindungen gemäß den Ansprüchen 1 bis 3, **dadurch gekennzeichnet, dass** das Epoxid gemäß Formel (III) mindestens eine Verbindung ist aus der Gruppe Ethylenglycoldiglycidylether, 1,2-Propandioldiglycidylether, 1,3-Butandioldiglycidylether, 1,4-Butandioldiglycidylether, Neopentylglykoldiglycidylether, Cyclohexandimethanoldiglycidylether, Diethylenglykoldiglycidylether, Dipropylenglykoldiglycidylether, Polyethyleneglykoldiglycidylether, Polypropylenglykoldiglycidylether, Glycerindiglycidylether, Resorcindiglycidylether, 2,2-Bis[4-(glycidyloxy)phenyl]propan, Bis(4-glycidyloxyphenyl)-methan, Bisphenol-A-propoxylat (1-PO/phenol)diglycidylether, 1,2-Cyclohexandicarbonsäure-diglycidylester.

5. Verbindungen gemäß den Ansprüchen 1 bis 4, **dadurch gekennzeichnet, dass** das Epoxid gemäß Formel (IV) mindestens eine Verbindung ist aus der Gruppe Trimethylolpropantriglycidylether, Triphenylolmethantriglycidylether, bzw. Mischungen der Verbindungen.

6. Aminoalkohole der allgemeinen Formeln (V) und (VI) 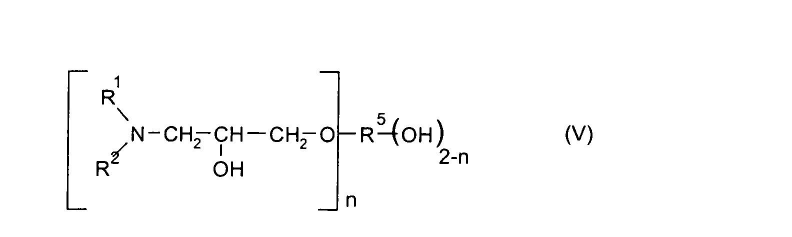 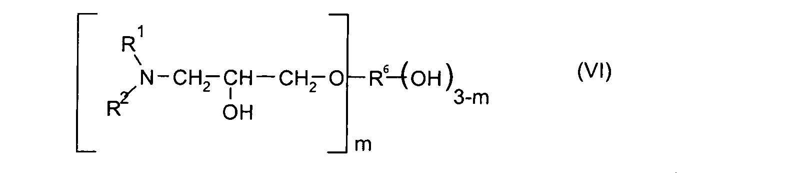 worin
R¹, R² die oben angegebene Bedeutung haben, und unabhängig voneinander vorzugsweise gegebenenfalls verzweigte Alkylenreste mit 1 bis 10 C-Atomen, und/oder R³-X- (CH₂)ₐ- worin R³, X, a die oben angegebene Bedeutung haben, R³ vorzugsweise ein gegebenenfalls verzweigter Alkylrest mit 1 bis 10 C-Atomen und X = -(O)C-NH- und a = 3 ist,
R⁵ und R⁶ die oben angegebene Bedeutung haben, und vorzugsweise unabhängig voneinander gegebenenfalls verzweigte Alkylenreste mit 1 bis 10 C-Atomen, und
n 1 bis 2,
m 1 bis 3 bedeuten.

7. Verwendung der Aminoalkohole gemäß den Ansprüchen 1 bis 6 als Oberflächenspannung reduzierende und gleichzeitig die Bildung/Neubildung von Schaum über einen langen Zeitraum effektiv inhibierende Additive in wässrigen Formulierungen.

8. Verwendung der Aminoalkohole gemäß Anspruch 7 zur Herstellung von Oberflächenbeschichtungen, Anstrichmitteln, Druckfarben oder Lacken.

9. Wässrige Formulierungen enthaltend mindestens einen Aminoalkohol der Ansprüche 1 bis 6.
